# EUROPEAN PATENT SPECIFICATION

(11) **EP 3 346 918 B1**
(45) Date of publication and mention of the grant of the patent: **30.10.2019**
(21) Application number: 16843897.6
(22) Date of filing: 16.08.2016
(51) Int. Cl.: A61B 5/05, G01R 33/035

(54) **MAGNETISM MEASURING APPARATUS**
MAGNETISMUSMESSVORRICHTUNG
APPAREIL DE MESURE DU MAGNÉTISME

(30) Priority: 10.09.2015 JP 2015178756; 08.07.2016 JP 2016136182
(43) Date of publication of application: 18.07.2018
(73) Proprietor: Ricoh Company, Ltd., Tokyo 143-8555 (JP)
(72) Inventor: YAMAGATA, Hideaki, Tokyo 143-8555 (JP)
(74) Representative: White, Duncan Rohan
(86) International application number: PCT/JP2016/003746
(87) International publication number: WO 2017/043024

(56) References cited:
- JP-A- H 114 814
- JP-A- H03 272 738
- JP-A- H04 319 334
- JP-A- H10 295 662
- JP-A- 2006 304 851
- US-A- 5 794 620
- US-A1- 2013 165 766

## Description

The present disclosure relates to a magnetism measuring apparatus.

### [Background Art]

Biomagnetic field measurement is known as a method of measuring a weak current generated in accordance with activities of nerves or muscles of a living body as magnetic fields outside the living body. Regarding biomagnetic field measurement, a system using a superconducting quantum interference device (SQUID) sensor array has been developed, and the measurement is actually performed as a magnetoencephalography or a magnetocardiography.

As a specific apparatus, for example, a magnetism measuring apparatus is disclosed in which a sensor array of multiple superconducting magnetic sensors is mounted on the inner surface at the distal portion of a sensor cylinder. In this magnetism measuring apparatus, as a superconducting magnetic sensor, a three-axis SQUID sensor is used in which a cylindrical block is equipped with three SQUIDs. The triaxial SQUID sensors are arranged in many directions to form a sensor array (e.g., PTL 1).

US-A-5794620 discloses a method for reconstructing the spatial current distribution in a biological object having a morphological structure, at least one component of the magnetic field produced by the current sources being measured at a number of points outside the object, after which the current distribution at the volume elements situated within the object is reconstructed from the measuring values. In order to improve the accuracy of reconstruction, in a representation containing the morphological structure of the object the surfaces are specified on which the current sources are presumably present, the reconstruction being limited to the volume elements which are situated on these surfaces.

JP-H-04319334 discloses, at a multi-channel living body magnetic imaging device in which the distribution of a faint magnetic field generated from a living body is measured by using a SQUID that is a magnetic sensor, and a living body inside active electric current location is surmised from the measured data, and its distribution is conducted with imaging; a living body electric current source position is surmised by using a neural network. At this time, the analogue voltage or digital signal of an SQUID magnetic flux meter which is proportional to a received magnetic field strength of each pick up coil is used as the input of the neural network, and as the output of the network, analogue voltage proportional to mutually vertical directional positions from a reference point where the electric current source is and to the 3 directional components of electric current intensity, or electric current density at two-dimensional and three-dimensional dispersion points, is outputted and displayed at a display.

JP-H-03272738 discloses multi-sliced images of the brain of a testee are photographed by a MRI device 5, and the picture of his brain surface configuration is also taken, so that these data are inputted into a computer 3. In the second place, brain magnetic fields at the respective measuring points of the brain are measured by a SQUID sensor 1. Three dimensional positions and directions in a head coordinate system are inputted into the computer 3, so that the coordinates of measured points in the head coordinate system are thereby obtained by way of the coordinate of a oscillator. After the positional relation of the measured points has been made clear, the magnetic field of the brain is measured by the SQUID sensor 1, the result is then inputted into the computer 3 via a data collecting device 2 so that current dipoles thereby work out. Obtained electric current dipoles and sensors are projected onto a cerebral cortex approximate model so as to be displayed on the image of the brain surface configuration.

### [Summary of Invention]

### [TECHNICAL PROBLEM]

An object of the present disclosure is to provide a magnetism measuring apparatus capable of increasing signal processing speed without lowering performance.

### [SOLUTION TO PROBLEM]

The invention provides a magnetism measuring apparatus as claimed in claim 1.

### [ADVANTAGEOUS EFFECTS OF INVENTION]

According to an embodiment, it is possible to provide a magnetism measuring apparatus capable of improving signal processing speed without lowering performance.

### [Brief Description of Drawings]

Fig. 1 is a drawing illustrating a spinal cord evoked magnetic field measuring system.
Fig. 2 is a drawing in which a vicinity of an electrode attached to a subject is magnified.

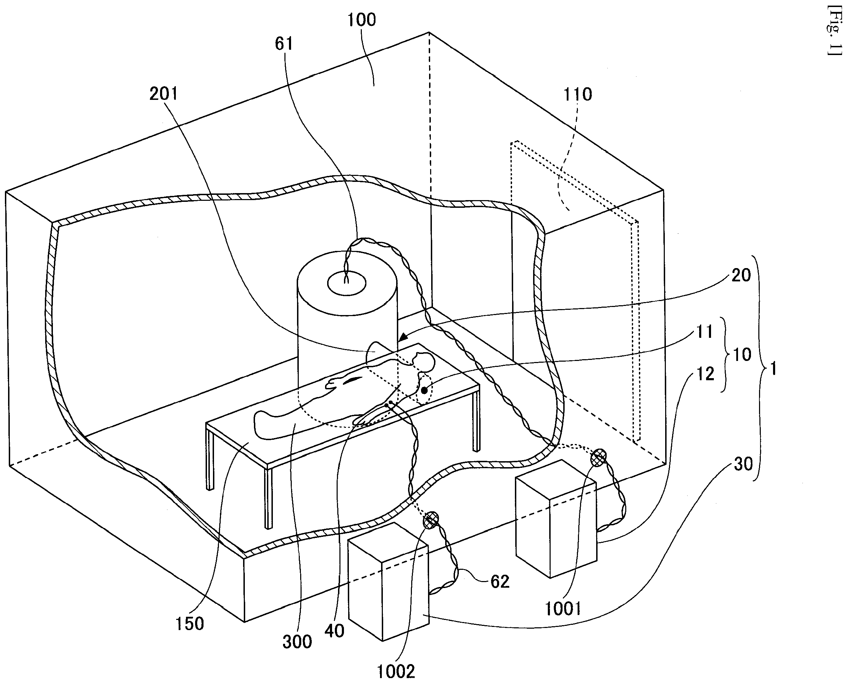

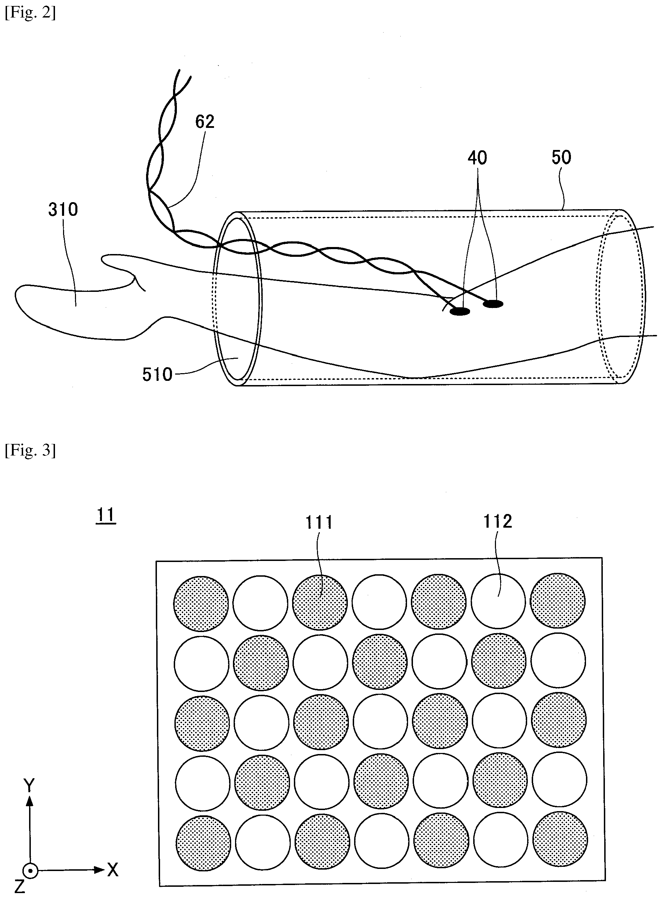

Referring to Fig. 1 and Fig. 2, the spinal cord evoked magnetic field measuring system 1 includes as main elements a magnetism measuring apparatus 10, a cryogenic container 20, and an electrical stimulation apparatus 30. The magnetism measuring apparatus 10 includes a SQUID sensor array 11 and a signal processing unit 12.

A part of the spinal cord evoked magnetic field measuring system 1 is arranged in a magnetism shielded room 100. The magnetism shielded room 100 is used in order to measure a spinal cord evoked magnetic field which is a weak magnetic field that a living body generates. The magnetism shielded room 100 can be constructed by, for example, laminating plates made of permalloy, etc., which is a high magnetic permeability material, and plates made of conductors such as copper and aluminum.

The magnetism shielded room 100 has, for example, an internal space about 2.5 m by 3.0 m by 2.5 m, and includes a door 110 which enables transporting equipment and instruments, and enables people going in and out. Similar to other parts of the magnetism shielded room 100, the door 110 can be constructed by laminating plates made of permalloy, etc., which is a high magnetic permeability material, and plates made of conductors such as copper and aluminum.

It should be noted that, in this specification, the high magnetic permeability material refers to a material whose relative permeability is greater than 1000. As such materials, other than the permalloy, a simple substance of iron, nickel, or cobalt, an alloy of iron, nickel, or cobalt (including amorphous alloy, powder, nanoparticles), ferrite, etc., can be listed.

In the following, the spinal cord evoked magnetic field measuring system 1 and its peripheral parts will be described in detail. In the magnetism shielded room 100, a bed 150 is arranged. Further, in the magnetism shielded room 100, the cryogenic container 20 is arranged, and a signal line 61 used for measurement, control, etc., is attached to the cryogenic container 20. The signal line 61 includes a twisted cable, etc., for reducing magnetic field noise, and is, through a hole 1001 opened in the magnetism shielded room 100, pulled out of the magnetism shielded room 100, and connected to a signal processing unit 12 included in the magnetism measuring apparatus 10.

In the measurement using the spinal cord evoked magnetic field measuring system 1, a subject 300 lies on his/her back in the bed 150 in the magnetism shielded room 100, and the spinal cord evoked magnetic field measurement is performed while the subject 300 is in a resting state. By performing the measurement while the subject 300 is in a resting state, it is possible, not only to reduce the burden on the subject 300, but also to reduce a positional gap between the subject 300 and the measuring apparatus due to unnecessary movement of the subject 300, and reduce magnetic field noise, etc., from muscles generated by muscle tension.

The cryogenic container 20 is also referred to as a dewar, and retains liquid helium necessary for a cryogenic operation of the SQUID sensor array 11 which detects magnetic fields generated from a living body. The cryogenic container 20 includes, for example, a protrusion unit 201 adapted to the spinal evoked magnetic field measurement, and the SQUID sensor array 11 is arranged within the liquid helium inside the protrusion unit 201.

By having the protrusion unit 201, inside of which the SQUID sensor array 11 is arranged, get closer to the cervical spine of the subject 300 from lower side of the subject who is lying on his/her back in the bed 150, it is possible to perform the spinal cord evoked magnetic field measurement while having the SQUID sensor array 11 closer to the measurement region.

When measuring the spinal cord evoked magnetic field, it is necessary to intentionally provoke nervous activities by using an electrical stimulus. Therefore, an electrode 40 is attached to a part 310 of a body of the subject 300, and an electrical stimulus is applied. The electrode 40 includes an anode and a cathode, and is attached onto a part of the skin where a signal is efficiently applied to median nerves of elbow joints and knee joints of the subject 300.

A signal line 62 for transmitting the stimulus is attached to the electrode 40. The signal line 62 includes a twisted cable, etc., for reducing magnetic field noise. The signal line 62 is, through a hole 1002 opened in the magnetism shielded room 100, pulled out of the magnetism shielded room 100, and connected to the electrical stimulation apparatus 30 arranged out of the magnetism shielded room 100.

In order to provoke nervous activities of the subject 300, the electrical stimulation apparatus 30 can cause a pulse-shaped current to flow between the anode and the cathode of the electrode 40. Regarding the electrical stimulus at the time of measuring spinal cord evoked magnetic fields, for example, a pulse current of about a few mA is applied at a few Hz. Magnetic fields from the spinal cord evoked by the electrical stimulus are detected by the SQUID sensor array 11.

In the spinal cord evoked magnetic field measuring system 1, the current itself used for electrical stimulus at the time of measuring spinal cord evoked magnetic fields is magnetic field noise. Specifically, the magnetic field, generated by the pulse current which flows in the signal line 62 from the electrical stimulation apparatus 30 to the electrode 40 and flows between the anode and the cathode of the electrode 40, goes into the SQUID sensor array 11 and becomes noise.

The noise generated by the signal line 62 is reduced by use of twisted cable and optical transmission. However, the noise generated by the pulse current flowing between the anode and the cathode of the electrode 40 cannot be reduced by the use of the twisted cable and the optical transmission. Therefore, in order to reduce the noise generated by the pulse current used for an electrical stimulus and to measure the spinal cord evoked magnetic field more accurately, a magnetism shielding cover 50 is used.

The magnetism shielding cover 50 can be made of, for example, high magnetic permeability material such as permalloy, etc. The higher magnetic permeability of which a material is made is used for the magnetism shielding cover 50, the higher will be the magnetism shielding effect. The magnetism shielding cover 50 has a space 510 in which the part 310 of the subject 300 is held.

The magnetism shielding cover 50 is grounded by a ground wire. It is preferable that the shape of the magnetism shielding cover 50 be cylindrical in order to reduce the magnetic field generated by the pulse current flowing between the anode and the cathode of the electrode 40. Further, it is preferable that the magnetism shielding cover 50 be fixed to the bed 150, etc., so that the relative positional relationship between the magnetism shielding cover 50 and the SQUID sensor array 11 is constant.

### (Magnetism measuring apparatus)

Next, the magnetism measuring apparatus 10 will be described in detail. Fig. 3 is a plan view illustrating an example of a SQUID sensor array according to an embodiment. As illustrated in Fig. 3, the SQUID sensor array 11 includes triaxial sensors 111 which detect magnetic field components in three directions, and uniaxial sensors 112 which detect a magnetic field component in one direction. In the SQUID sensor array 11, the triaxial sensors 111 and the uniaxial sensors 112 are arranged alternatingly in the form of a matrix.

Here, as an example, thirty five sensors are arranged, which is not a limitation. Any number of sensors may be arranged. It should be noted that the triaxial sensors 111 are representative examples of the first sensors according to an embodiment, and the uniaxial sensors 112 are representative examples of the second sensors according to an embodiment.

The triaxial sensors 111 are configured in such a way that the triaxial sensors 111 can detect at the same time magnetic fields in three directions including X direction which is a longer side direction of a measurement surface (surface illustrated in Fig. 3) of the SQUID sensor array 11, Y direction which is a shorter side direction of the measurement surface, and a direction perpendicular to the measurement surface (XY plane). The triaxial sensors 111 can be realized by, for example, having one coaxial difference pick-up coil (for detecting magnetic fields in Z direction) and two planar difference pick-up coils (for detecting magnetic fields in XY directions) arranged perpendicular to each other in a cylindrical member.

The uniaxial sensors 112 are configured in such a way that the uniaxial sensors 112 can detect only magnetic fields in Z direction perpendicular to the measurement surface (XY plane) of the SQUID sensor array 11. The uniaxial sensors 112 can be realized by, for example, having one coaxial difference pick-up coil (for detecting magnetic fields in Z direction) in a cylindrical member.

It should be noted that it is possible to configure the uniaxial sensors 112 in such a way that the uniaxial sensors 112 can detect only magnetic fields in X direction, or that the uniaxial sensors 112 can detect only magnetic fields in Y direction. However, it is experimentally confirmed that the most preferable result is obtained in the current source reconstruction as described below when the uniaxial sensors 112 are configured in such a way that the uniaxial sensors 112 can detect only magnetic fields in Z direction.

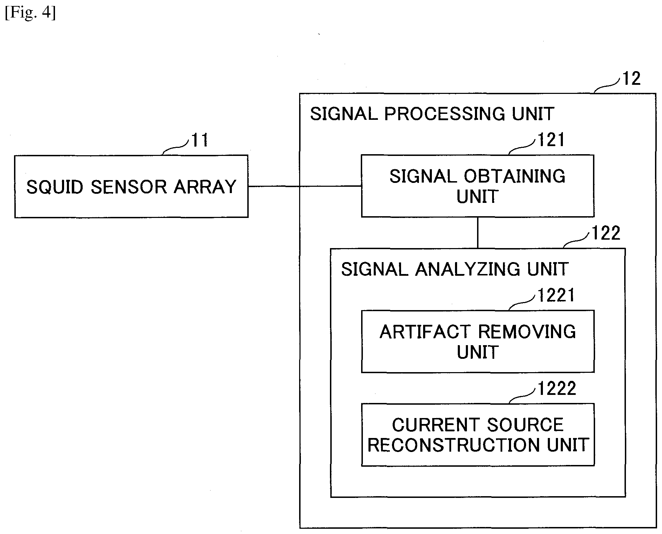

Fig. 4 is a drawing illustrating an example of a functional block diagram of a magnetism measuring apparatus according to an embodiment. As illustrated in Fig. 4, a magnetic field signal output from the SQUID sensor array 11 is transmitted to the signal processing unit 12. The signal processing unit 12 includes a signal obtaining unit 121 and a signal analyzing unit 122. Further, the signal analyzing unit 122 includes an artifact removing unit 1221 and a current source reconstruction unit 1222.

The signal obtaining unit 121 converts the magnetic field signal output from the SQUID sensor array 11 to a form available to the signal analyzing unit 122. For example, the analog magnetic field signal output from the SQUID sensor array 11 is converted to a digital magnetic field signal by the signal obtaining unit 121.

The artifact removing unit 1221 of the signal analyzing unit 122 removes artifacts based on the magnetic field signal obtained from the SQUID sensor array 11. In other words, the artifact removing unit 1221 removes the artifacts based on the digital magnetic field signal transmitted from the signal obtaining unit 121.

Further, the current source reconstruction unit 1222 of the signal analyzing unit 122 reconstructs a current source of a current flowing inside of a living body based on the magnetic field signal obtained from the SQUID sensor array 11. In other words, the current source reconstruction unit 1222 reconstructs a current source of a current flowing inside of a living body based on the digital magnetic field signal in which the artifacts have been removed.

It should be noted that the signal analyzing unit 122 can include, for example, a central processing unit (CPU), a read only memory (ROM), a main memory, etc. In this case, various functions of the signal analyzing unit 122 can be realized by having a program stored in the ROM, etc., read into the main memory and executed by the CPU.

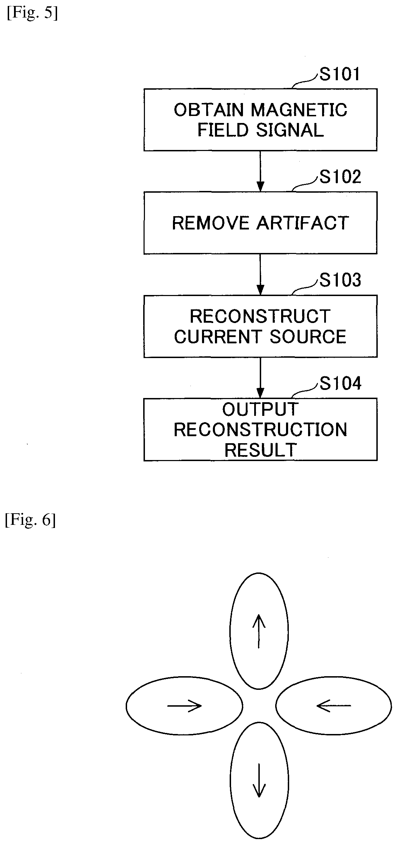

Fig. 5 is a flowchart illustrating an example of processing by the signal analyzing unit 122 according to an embodiment. As illustrated in Fig. 5, first, in step S101, the artifact removing unit 1221 of the signal analyzing unit 122 obtains via the signal obtaining unit 121 the magnetic field signal (referred to as a magnetic field signal A) detected by the SQUID sensor array 11 when an electrical stimulus is applied by the electrode 40. It should be noted that the artifact removing unit 122 may perform processes of averaging, baseline correction, moving average, etc., for the magnetic field signal A.

Next, in step S102, the artifact removing unit 1221 of the signal analyzing unit 122 removes artifacts from the magnetic field signal A. Here, the artifacts mean a noise component, other than the target, which is mixed with the obtained magnetic field signal.

In order to remove artifacts, for example, an artifact measurement electrode for measuring only (ideally) artifacts is arranged in the vicinity of the electrode 40 illustrated in Fig. 2. Further, the artifact removing unit 1221 obtains via the signal obtaining unit 121 a magnetic field signal (referred to as a magnetic field signal B), for removing artifacts, detected by the SQUID sensor array 11 when the electrical stimulus is applied by the electrode for artifact measurement. Further, the artifact removing unit 1221 removes artifacts by applying a method of matrix singular value decomposition (SVD) for the magnetic field signals A and B. At this time, it is necessary to detect magnetic fields in multiple directions.

Further, a method has been also investigated in which artifacts are removed only from the magnetic field signal A without including the electrode for artifact measurement (without obtaining the magnetic field signal B). In this method, artifacts are removed by performing a matrix operation using multiple magnetic field signals A measured at different times.

It should be noted that a detailed method of removing artifacts is disclosed in, for example, "Removal of Stimulus-Induced Artifacts in Functional Spinal Cord Imaging" (Taishi Watanabe, Yuya Kawabata, Dai Ukegawa, Shigenori Kawabata, Yoshiaki Adachi Member IEEE, Kensuke Sekihara, Fellow IEEE).

Next, in step S103, the current source reconstruction unit 1222 of the signal analyzing unit 122 performs reconstruction of a current source which is a magnetic field generating source based on the digital magnetic field signal in which artifacts have been removed.

The reconstruction of the current source can be performed by, for example, using a spatial filter method. Specifically, weight matrix W(r) for current density of a position (r) for the obtained magnetic field signal b(t) is defined as W(r)=G-1L(r) by using a Gram matrix G and a Lead Field matrix L. The Lead Field matrix can be calculated according to a positional relationship between a sensor and a reconstruction surface. The Gram matrix is used for defining characteristics of a filter, and various kinds of Gram matrices have been proposed. Further, by applying weight matrix W(r) to the magnetic field signal b(t), reconstructed current density s(r, t) can be obtained.

It should be noted that the detailed method of current source reconstruction is disclosed in, for example, "Array-Gain Constraint Minimum-Norm Spatial Filter With Recursively Updated Gram Matrix For Biomagnetic Source Imaging" (Isamu Kumihashi and Kensuke Sekihara, Fellow, IEEE), and "Functional Imaging of Spinal Cord Electrical Activity From Its Evoked Magnetic Field" (Tomoya Sato, Yoshiaki Adachi, Member, IEEE, Masaki Tomori, Senichi Ishii, Shigenori Kawabata, and Kensuke Sekihara, Fellow, IEEE).

Next, in step S104, the current source reconstruction unit 1222 of the signal analyzing unit 122 outputs a result of current source reconstruction to a display, etc. It is ideal that the current sources of currents flowing in nerve cells in a living body are reconstructed as illustrated in Fig. 6. In Fig. 6, a direction of an arrow indicates a current flow direction, and a length of an arrow indicates magnitude of a current. As illustrated in Fig. 6, it is ideal that peaks of four currents including two outgoing currents (up and down in Fig. 6) and two returning currents (right and left in Fig. 6) look uniform.

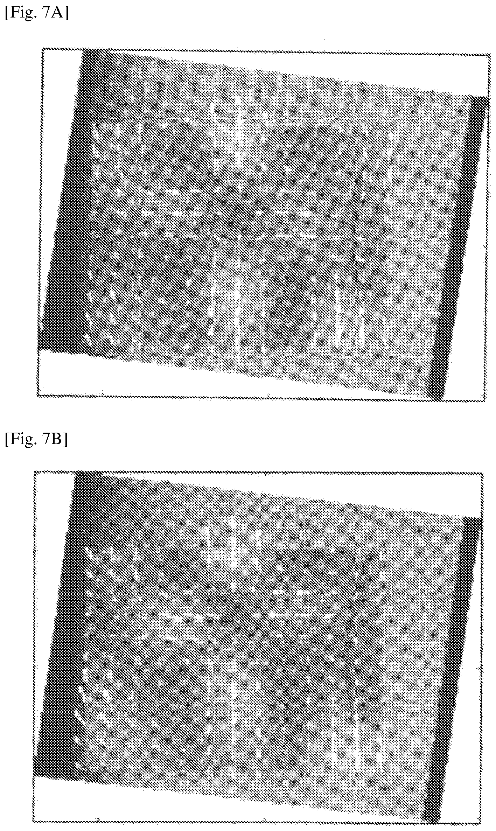

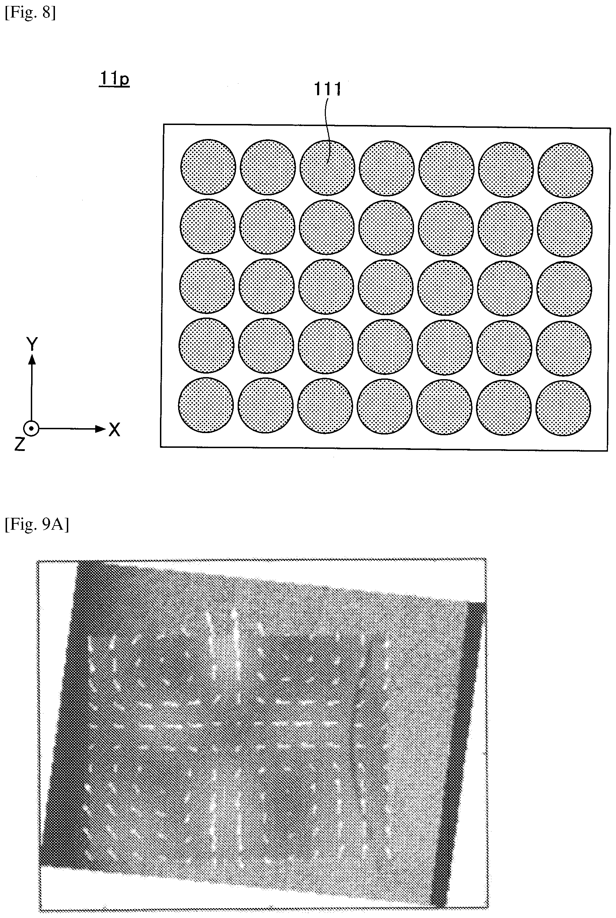

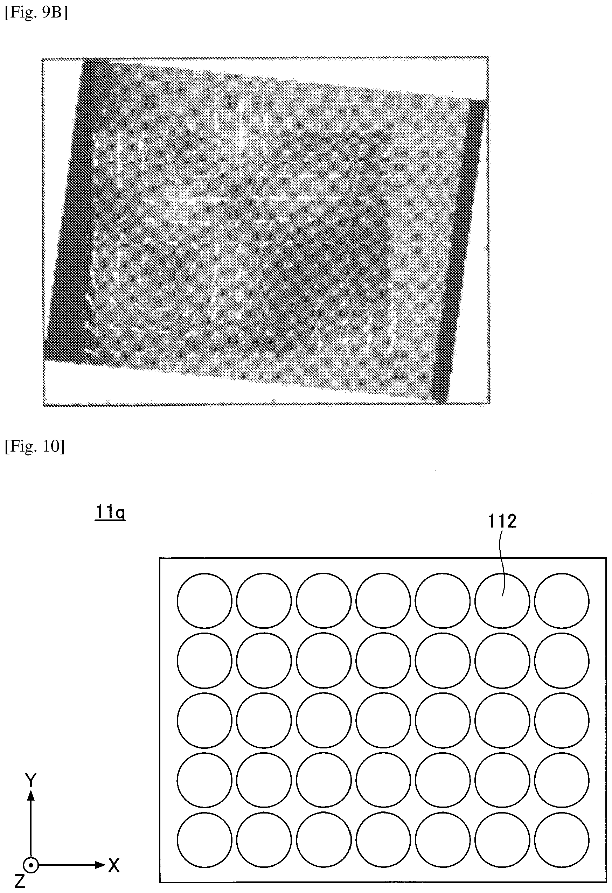

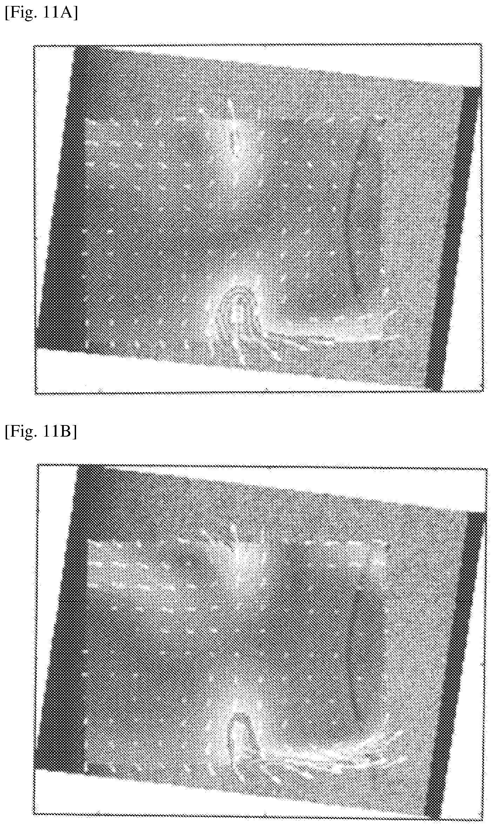

Fig. 7 is a drawing illustrating an example in which current sources are reconstructed by using the sensor array of Fig. 3. As illustrated in Fig. 7A and Fig. 7B, in the case where the SQUID sensor array 11 is used, current sources are reconstructed in a manner very close to Fig. 6, and thus, it can be seen that the SQUID sensor array 11 demonstrates sufficient performance. It should be noted that Fig. 7A illustrates data after about 10 ms from the start of the stimulus, and Fig. 7B illustrates data after 0.025 ms from Fig. 7A (the same can be applied to Figs. 9A and 9B, and Figs. 11A and 11B, which will be described later).

### (Comparison)

Next, unique effects of the magnetism measuring apparatus 10 will be described by including comparative examples. Fig. 8 is a plan view illustrating a SQUID sensor array according to a comparative example 1. As illustrated in Fig. 8, in a SQUID sensor array 11p according to the comparative example 1, only triaxial sensors 111 are arranged in the form of a matrix.

Fig. 9A and Fig. 9B are drawings illustrating examples in which current sources are reconstructed by using the sensor array of Fig. 8. As illustrated in Fig. 9A and Fig. 9B, in the case where the SQUID sensor array 11p is used, current sources are reconstructed in a manner very close to Fig. 6, and thus, it can be seen that the SQUID sensor array 11p demonstrates sufficient performance.

Fig. 10 is a plan view illustrating a SQUID sensor array according to a comparative example 2. As illustrated in Fig. 10, in a SQUID sensor array 11q according to the comparative example 2, only uniaxial sensors 112 are arranged in the form of a matrix.

Fig. 11A and Fig. 11B are drawings illustrating examples in which current sources are reconstructed by using the sensor array of Fig. 10. As illustrated in Fig. 11A and Fig. 11B, in the case where the SQUID sensor array 11q is used, current sources are reconstructed in the form very different from Fig. 6, and thus, it can be seen that sufficient performance cannot be obtained from the SQUID sensor array 11q.

In this case, it can be considered that insufficient artifact removal may be a cause of insufficient performance. In other words, because it is necessary to detect magnetic fields in multiple directions in order to remove artifacts, it is preferable to include triaxial sensors 111 and it can be considered that including only uniaxial sensors 112 is insufficient.

As described above, when an SQUID sensor array includes only uniaxial sensors, the SQUID sensor array cannot perform current source reconstruction sufficiently. On the other hand, when an SQUID sensor array includes only triaxial sensors, there may be no problem in terms of performance, but, as described in the related art, there occurs a processing time problem. The detail will be described below.

In general, when calculating a signal source by using many signals, many matrix operations are used. For example, in the artifact removal, a process is included in which the singular value is calculated for an matrix of a size of the number of sensors. When it is assumed that the number of sensors is M, the singular value is calculated for a M×M matrix by using an SVD method.

At this time, the amount of calculation will be O(M2) even when a fast method is used, and will be O(M3) when a general method is used. In other words, when the number of sensors M is 1/2, the amount of calculation will be reduced to 1/4 to 1/8 (processing speed will be improved by 4 to 8 times). It should be noted that the fast method is disclosed in, for example, "Recent Developments of the mdLVs Algorithm for Computing Matrix Singular Values" (Institute for Mathematical Sciences Kokyu proceedings, No. 1594 vol., 2008, 136-148).

Further, in the current source reconstruction, an inverse matrix of a Gram matrix G is calculated. When the number of reconstruction points is N, G is a M×N matrix. Therefore, similar to the case of artifact removal, when the number of sensors M is 1/2, the amount of calculation will be reduced to 1/4 to 1/8 (processing speed will be improved by 4 to 8 times).

As described above, in general, in a calculation of the matrix to each other, the amount of calculation will be O(M2) to O(M3), and thus, processing speed can be significantly improved by reducing the number of sensors.

It should be noted that by using triaxial sensors, it becomes possible to detect magnetic fields in multiple directions, and performance of artifact removal is greatly improved. However, in the artifact removal, high sensor density is not necessarily needed. It is important that sensors are arranged in wide range with an appropriate density.

On the other hand, in the current source reconstruction, it is not necessarily needed that components in three directions are measured. It is possible to perform current source reconstruction by using only uniaxial sensors in Z direction. However, it is necessary to increase sensor density to some extent in order to identify a current source position in detail.

In other words, in order to increase resolution of current source reconstruction, it is necessary to increase sensor density to some extent, but it is sufficient to use only uniaxial sensors. On the other hand, in view of the artifact removal, it is necessary to use triaxial sensors, but high density sensor arrangement is not necessarily needed.

Therefore, in the SQUID sensor array 11, minimum number of triaxial sensors necessary for artifact removal are arranged, and other sensors are uniaxial sensors. As is obvious from the comparison between Figs. 7A, 7B and Figs. 9A, 9B, compared with the case where the SQUID sensor array 11p (refer to Fig. 8) is used, the performance is not decreased even when the SQUID sensor array 11 (refer to Fig. 3) is used.

Further, by using the SQUID sensor array 11, compared with the case where the SQUID sensor array 11p is used, signal processing speed is greatly improved. Specifically, the number of signals in Fig. 3 is triaxial sensors 111×18 + uniaxial sensors 112×17 = 71. On the other hand, the number of signals in Fig. 8 is triaxial sensors 111×35 = 105.

In other words, when the SQUID sensor array 11 is used, the amount of calculation is O(M2) to O(M3), and the ratio of the number of signals compared with the case where the SQUID sensor array 11p is used is 71/105. Therefore, processing time (the amount of calculation) is 0.45 to 0.31, and thus, it is possible to make processing time less than half (processing speed more than double).

It is necessary that calculation of artifact removal and current source reconstruction performed by the signal processing unit 12 are performed within limited time from when magnetic field signals are obtained till an examination is performed. From the above point of view, improvement in the processing speed will be a great advantage when building a spinal cord evoked magnetic field measuring system 1, etc.

Further, if there is room for processing time, it is possible to increase the number of signals. It is possible to increase the number of sensors by double or triple in the same processing time. It is possible to improve detection accuracy of current source position by having more (thinner) sensors arranged on the same size of the sensor array. Further, by having more sensors arranged on the wider size of sensor array, current sources in a wider area can be searched.

Further, due to the complexity of the structure, compared with the uniaxial sensors 112, yield of triaxial sensors 111 is very bad. As a result, the triaxial sensors 111 are expensive compared with the uniaxial sensors 112. In other words, when sensor density of the triaxial sensors 111 is increased for higher resolution current source reconstruction, the price of the sensor array becomes very expensive.

By using the SQUID sensor array 11, the use number of the triaxial sensors 111 is reduced by about a half compared with the case where the SQUID sensor array 11p is used, and thus, the SQUID sensor array 11 has a big advantage in terms of cost reduction.

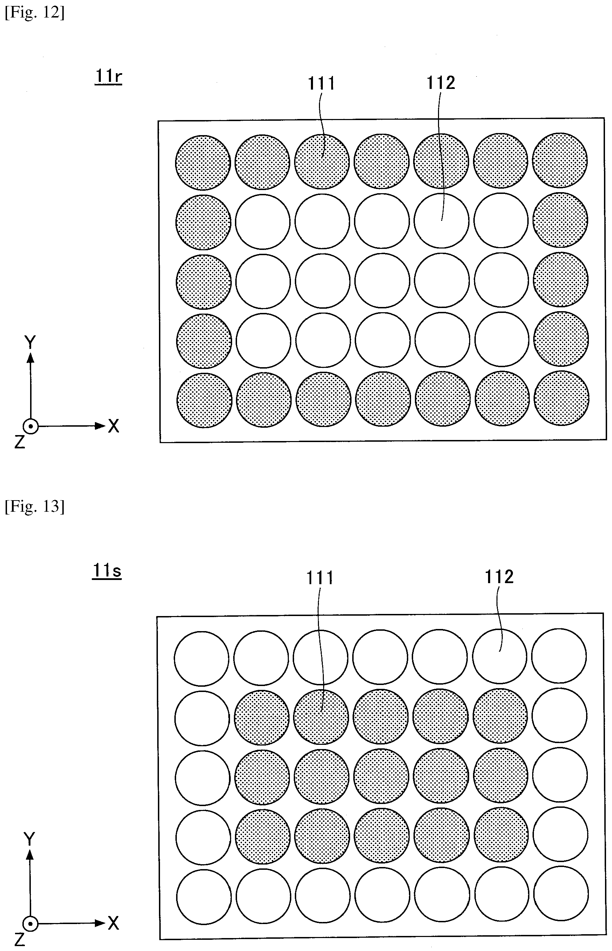

It should be noted that the arrangement of the triaxial sensors 111 and the uniaxial sensors 112 can be appropriately determined. For example, triaxial sensors 111 may be arranged only around as in a SQUID sensor array 11r illustrated in Fig. 12, or triaxial sensors 111 may be arranged only in the center as in a SQUID sensor array 11s illustrated in Fig. 13.

However, in order to obtain good performance, it is preferable that the triaxial sensors 111 and the uniaxial sensors 112 are arranged in such a way that, in an area where the triaxial sensors 111 and the uniaxial sensors 112 are arranged, any row or any column includes triaxial sensors 111 and uniaxial sensors 112.

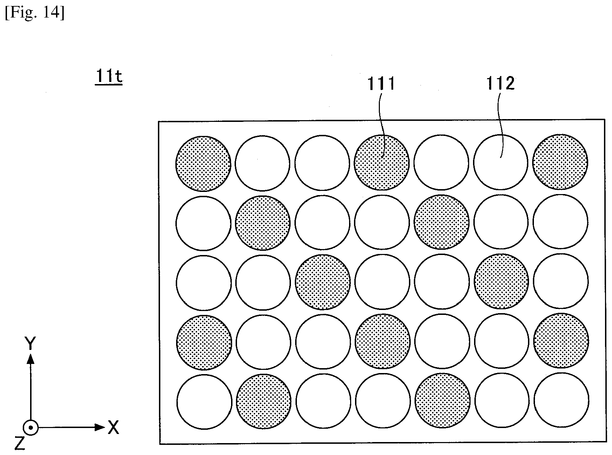

For example, as shown in a SQUID sensor array 11t illustrated in Fig. 14, in each column and each row in an area where triaxial sensors 111 and uniaxial sensors 112 are arranged, two uniaxial sensors are arranged between adjacent triaxial sensors 111. Alternatively, in each column and each row in an area where triaxial sensors 111 and uniaxial sensors 112 are arranged, three or more uniaxial sensors may be arranged between adjacent triaxial sensors 111.

In order to obtain further better performance, as illustrated in Fig. 3, in an area where the triaxial sensors 111 and the uniaxial sensors 112 are arranged, it is preferable to arrange triaxial sensors 111 as evenly (at regular intervals) as possible.

It should be noted that it has been described in the above that uniaxial sensors are sufficient for improving resolution of current source reconstruction; triaxial sensors are needed from removing artifacts point of view; and thus, it is preferable to arrange both sensors mixed arbitrarily. However, it is possible to obtain a certain level of effect by using biaxial sensors (sensors which detect magnetic field components in any two directions of XYX directions).

For example, biaxial sensors and uniaxial sensors may be arbitrarily mixed and arranged, triaxial sensors and biaxial sensors may be arbitrarily mixed and arranged, or triaxial sensors, biaxial sensors, and uniaxial sensors may be arbitrarily mixed and arranged

In other words, it is possible to obtain a certain level of effect in removing artifacts, securing performance of current source reconstruction, and improving signal processing speed, by at least arranging a mixture of first sensors which detect magnetic field components in many directions and second sensors which detect magnetic field components in directions fewer than the first sensors.

The preferred embodiments have been described. However, the embodiments are not limited to as described above, and various modifications and replacements may be applied to the above embodiments without departing from the scope of claims.

For example, in an embodiment described above, in a magnetism measuring apparatus, an example is illustrated in which a SQUID sensor is used for forming a sensor array, which is not limited to use a SQUID sensor. In a magnetism measuring apparatus according to an embodiment, as a sensor for forming a sensor array, for example, an atomic magnetometer (AMM element), a magnetoresistive element (MR element), a magnetic impedance element (MI sensor), etc., may be used. Alternatively, in a magnetism measuring apparatus according to an embodiment, a mixture of the above sensors may be arranged.

Further, in the above, an example is illustrated in which a magnetism measuring apparatus according to an embodiment is used in, but is not limited to, a spinal cord evoked magnetic field measuring system (spinal cord meter) for detecting a current flowing in nerves running in the spinal cord as a magnetic field. A magnetism measuring apparatus according to an embodiment may be used, for example, in a magnetoencephalography or a magnetocardiography.

The present application is based on and claims the benefit of priority of Japanese Priority Application No. 2015-178756 filed on September 10, 2015, and Japanese Priority Application No. 2016-136182 filed on July 8, 2016, the entire contents of which are hereby incorporated by reference.

### Reference Signs List

- 1: Spinal cord evoked magnetic field measuring system
- 10: Magnetism measuring apparatus
- 11: SQUID sensor array
- 12: Signal processing unit
- 20: Cryogenic container
- 30: Electrical stimulation apparatus
- 40: Electrode
- 50: Magnetism shielding cover
- 61, 62: Signal line
- 100: Magnetism shielded room
- 110: Door
- 111: Triaxial sensor
- 112: Uniaxial sensor
- 121: Signal obtaining unit
- 122: Signal analyzing unit
- 150: Bed
- 201: Protrusion unit
- 300: Subject
- 310: A part of a body of a subject
- 510: Space
- 1001, 1002: Hole
- 1221: Artifact removing unit
- 1222: Current source reconstruction unit

### Citation List

### Patent Literature

[PTL 1] Japan Patent No. 4834076

## Claims

1. A magnetism measuring apparatus comprising:
a sensor array (11) configured to detect a magnetic field generated from a living body (300), wherein the sensor array (11) includes first sensors (111) for detecting magnetic field components in many directions and second sensors (112) for detecting magnetic field components in directions fewer than the first sensors (111); and
**characterized by** a current source reconstruction unit (1222) configured to reconstruct a current source of a current flowing inside of the living body (300) based on a magnetic field signal obtained from the first and second sensors (111, 112) of the sensor array (11).

2. The magnetism measuring apparatus according to claim 1, wherein the first sensors (111) detect magnetic field components in three directions, and the second sensors (112) detect a magnetic field component in one direction.

3. The magnetism measuring apparatus according to claim 1 or 2, further comprising:
an artifact removing unit (1221) configured to remove artifacts based on the magnetic field signal obtained from the sensor array (11).

4. The magnetism measuring apparatus according to any one of claims 1 through 3, wherein the first sensors (111) are arranged at regular intervals in an area where the first sensors (111) and the second sensors (112) are arranged.

5. The magnetism measuring apparatus according to any one of claims 1 through 4, wherein, in an area where the first sensors (111) and the second sensors (112) are arranged, the first sensors (111) and the second sensors (112) are arranged in such a way that the first sensors (111) and the second sensors (112) are included in each row and each column.

6. The magnetism measuring apparatus according to any one of claims 1 through 5, wherein the second sensors (112) detect at least a magnetic field component in a direction perpendicular to a measurement surface of the sensor array (11).

7. The magnetism measuring apparatus according to claim 2, wherein the second sensors (112) detect the magnetic field component in a direction perpendicular to a measurement surface of the sensor array (11).

8. The magnetism measuring apparatus according to any one of claims 1 through 7, wherein at least one of the first sensors (111) and the second sensors (112) is a SQUID sensor.

9. The magnetism measuring apparatus according to any one of claims 1 through 8, wherein the current flows in nerves running in a spinal cord.

10. The magnetism measuring apparatus as claimed in any one preceding claim, wherein the sensors of the array are of the same type.

11. The magnetism measuring apparatus as claimed in claim 10, wherein the sensors are one of, SQUIDs, atomic magnetometers, magneto-resistive elements, or magnetic impedance elements.

## Patentansprüche

1. Magnetismusmessvorrichtung, umfassend:
eine Sensoranordnung (11), konfiguriert zum Detektieren eines von einem lebenden Körper (300) erzeugten Magnetfelds, wobei die Sensoranordnung (11) erste Sensoren (111) zum Detektieren von Magnetfeldkomponenten in vielen Richtungen und zweite Sensoren (112) zum Detektieren von Magnetfeldkomponenten in weniger Richtungen als die ersten Sensoren (111) enthält; und
**gekennzeichnet durch**
eine Stromquellen-Rekonstruktionseinheit (1222), konfiguriert zum Rekonstruieren einer Stromquelle eines Stroms, der im Inneren des lebenden Körpers (300) fließt, basierend auf einem von den ersten und zweiten Sensoren (111, 112) der Sensoranordnung (11) erhaltenen Magnetfeldsignal.

2. Magnetismusmessvorrichtung nach Anspruch 1, wobei die ersten Sensoren (111) Magnetfeldkomponenten in drei Richtungen detektieren und die zweiten Sensoren (112) eine Magnetfeldkomponente in einer Richtung detektieren.

3. Magnetismusmessvorrichtung nach Anspruch 1 oder 2, ferner umfassend:
eine Artefaktentfernungseinheit (1221), konfiguriert zum Entfernen von Artefakten basierend auf dem von der Sensoranordnung (11) erhaltenen Magnetfeldsignal.

4. Magnetismusmessvorrichtung nach einem der Ansprüche 1 bis 3, wobei die ersten Sensoren (111) in regelmäßigen Intervallen in einem Bereich angeordnet sind, in dem die ersten Sensoren (111) und die zweiten Sensoren (112) angeordnet sind.

5. Magnetismusmessvorrichtung nach einem der Ansprüche 1 bis 4, wobei in einem Bereich, in dem die ersten Sensoren (111) und die zweiten Sensoren (112) angeordnet sind, die ersten Sensoren (111) und die zweiten Sensoren (112) in einer derartigen Weise angeordnet sind, dass die ersten Sensoren (111) und die zweiten Sensoren (112) in jeder Reihe und jeder Spalten enthalten sind.

6. Magnetismusmessvorrichtung nach einem der Ansprüche 1 bis 5, wobei die zweiten Sensoren (112) mindestens eine Magnetfeldkomponente in einer Richtung senkrecht zu einer Messungsoberfläche der Sensoranordnung (11) detektieren.

7. Magnetismusmessvorrichtung nach Anspruch 2, wobei die zweiten Sensoren (112) die Magnetfeldkomponente in einer Richtung senkrecht zu einer Messungsoberfläche der Sensoranordnung (11) detektieren.

8. Magnetismusmessvorrichtung nach einem der Ansprüche 1 bis 7, wobei mindestens einer der ersten Sensoren (111) und der zweiten Sensoren (112) ein SQUID-Sensor ist.

9. Magnetismusmessvorrichtung nach einem der Ansprüche 1 bis 8, wobei der Strom in Nerven fließt, die in einem Rückenmark verlaufen.

10. Magnetismusmessvorrichtung nach einem der vorstehenden Ansprüche, wobei die Sensoren der Anordnung des gleichen Typs sind.

11. Magnetismusmessvorrichtung nach Anspruch 10, wobei die Sensoren eines von SQUIDs, atomischen Magnetometern, magnetoresistiven Elementen oder Magnetimpedanzelementen sind.

## Revendications

1. Appareil de mesure de magnétisme comprenant :
un réseau de capteurs (11) configurés pour détecter un champ magnétique produit par un organisme vivant (300), dans lequel le réseau de capteurs (11) inclut des premiers capteurs (111) pour détecter des composantes de champ magnétique dans plusieurs directions et des seconds capteurs (112) pour détecter des composantes de champ magnétique dans moins de directions que les premiers capteurs (111), et
**caractérisé par**
une unité de reconstitution de source de courant (1222) configurée pour reconstituer une source de courant d'un courant circulant à l'intérieur de l'organisme vivant (300) sur la base d'un signal de champ magnétique obtenu à partir des premier et second capteurs (111 , 112) du réseau de capteurs (11).

2. Appareil de mesure de magnétisme selon la revendication 1, dans lequel les premiers capteurs (111) détectent des composantes de champ magnétique dans trois directions, et les seconds capteurs (112) détectent une composante de champ magnétique dans une direction.

3. Appareil de mesure de magnétisme selon la revendication 1 ou 2, comprenant en outre :
une unité d'enlèvement d'artefacts (1221) configurée pour enlever des artefacts sur la base du signal de champ magnétique obtenu à partir du réseau de capteurs (11).

4. Appareil de mesure de magnétisme selon l'une quelconque des revendications 1 à 3, dans lequel les premiers capteurs (111) sont disposés à des intervalles réguliers dans une région où les premiers capteurs (111) et les seconds capteurs (112) sont agencés.

5. Appareil de mesure de magnétisme selon l'une quelconque des revendications 1 à 4, dans lequel, dans une région où les premiers capteurs (111) et les seconds capteurs (112) sont agencés, les premiers capteurs (111) et les seconds capteurs (112) sont agencés de telle manière que les premiers capteurs (111) et les seconds capteurs (112) sont inclus dans chaque rangée et dans chaque colonne.

6. Appareil de mesure de magnétisme selon l'une quelconque des revendications 1 à 5, dans lequel les seconds capteurs (112) détectent au moins une composante de champ magnétique dans une direction perpendiculaire à une surface de mesure du réseau de capteurs (11).

7. Appareil de mesure de magnétisme selon la revendication 2, dans lequel les seconds capteurs (112) détectent la composante de champ magnétique dans une direction perpendiculaire à une surface de mesure du réseau de capteurs (11).

8. Appareil de mesure de magnétisme selon l'une quelconque des revendications 1 à 7, dans lequel au moins un des premiers capteurs (111) et des seconds capteurs (112) est un capteur SQUID.

9. Appareil de mesure de magnétisme selon l'une quelconque des revendications 1 à 8, dans lequel le courant circule dans des nerfs passant dans une moelle épinière.

10. Appareil de mesure de magnétisme selon l'une quelconque des revendications précédentes, dans lequel les capteurs du réseau sont du même type.

11. Appareil de mesure de magnétisme selon la revendication 10, dans lequel les capteurs sont un des suivants : SQUID, magnétomètres atomiques, éléments magnéto-résistifs ou éléments d'impédance magnétique.
